# EUROPEAN PATENT APPLICATION

(11) **EP 4 759 254 A1**
(43) Date of publication of application: **17.06.2026**
(21) Application number: 23956624.3
(22) Date of filing: 29.11.2023
(51) Int. Cl.: A61B 18/00, A61M 25/10

(54) **METHOD FOR DETECTING BALLOON CATHETER ABUTMENT PRESSURE AND BALLOON CATHETER ABLATION SYSTEM**

(30) Priority: 23.10.2023 CN 202311367771
(71) Applicant: Enchannel Medical Guangzhou Inc., Guangzhou, Guangdong 510700 (CN)
(72) Inventor: HUANG, Long, Guangzhou, Guangdong 510700 (CN); MO, Zhidai, Guangzhou, Guangdong 510700 (CN); FENG, Jun, Guangzhou, Guangdong 510700 (CN)
(74) Representative: Manitz Finsterwald Patent- und Rechtsanwaltspartnerschaft mbB
(86) International application number: PCT/CN2023/135070
(87) International publication number: WO 2025/086387

(57) **Abstract**

The present disclosure relates to a method for detecting a balloon catheter contact pressure and a balloon catheter ablation system. The method comprises the following steps: determining, when the balloon catheter in an inflated state abuts against a target ablation tissue and undergoes axial compression and/or lateral deflection, an axial distance x between a proximal end and a distal end of a balloon in the balloon catheter and/or an angle α between a central axis of the proximal end and a central axis of the distal end of the balloon using a proximal magnetic sensor and a distal magnetic sensor; and determining an actual axial contact pressure and/or an actual lateral contact pressure by mapping the axial distance x and/or the angle α , based on a predefined first correspondence between the axial distance x and the axial contact pressure of the balloon catheter and/or a predefined second correspondence between the angle α and the lateral abutment pressure. Correspondingly, the balloon of the balloon electrode assembly can undergo axial deformation and/or lateral deflection. The present disclosure mainly solves the technical problem of detecting the contact pressure of a balloon catheter.

## Description

### TECHNICAL FIELD

The present disclosure relates to a method for detecting contact pressure of a balloon catheter and a balloon catheter ablation system.

### BACKGROUND OF THE DISCLOSURE

Ablation methods for treating arrhythmias include radiofrequency ablation and pulsed field ablation. Pulsed field ablation (PFA) is a novel technique for treating atrial fibrillation, which involves applying a strong electric field for a short duration to induce cellular electroporation, resulting in cell death. PFA can achieve greater ablation depth and a larger ablation range, and has been successfully established as a novel non-thermal ablation method for ablating cardiac tissue in arrhythmia treatment. Currently, research on pulsed field ablation primarily focuses on the "one-shot" design concept, i.e., forming a circumferential lesion in a single step to enable rapid isolation of the pulmonary veins. However, the pulmonary vein lesions responsible for atrial fibrillation are complex and diverse, such as the left common ostium or multiple small ostial branches. One-shot catheters cannot effectively achieve ablation isolation at pulmonary veins with anatomical variations. For such cases, point-by-point ablation offers a better alternative.

However, during point-by-point ablation, contact between the tissue and the balloon catheter occurs only at localized regions of the balloon electrode assembly. To ensure the ablation efficacy, feedback regarding the contact pressure between the catheter and the tissue is required to guide ablation maneuvers. If a pulsed field ablation catheter employs a strain gauge-based pressure sensor for contact pressure detection, it will occupy the proximal space of the catheter. However, in the case of balloon catheters, since an inflation tube for balloon filling is already present with the catheter, there is no available space for a strain gauge-based pressure sensor. Additionally, the addition of an additional pressure sensor will increase the cost of the device and the economic burden on patients.

### SUMMARY OF THE DISCLOSURE

The present disclosure provides a method for detecting contact pressure of a balloon catheter, and a balloon catheter ablation system. The disclosure primarily addresses technical problem associated with contact pressure detection in balloon catheters.

In a first aspect, the present disclosure provides a method for detecting contact pressure of a balloon catheter.

The method includes the following steps:
when an inflated balloon catheter contacts a tissue to be ablated and undergoes axial compressive deformation and/or lateral deflection, detecting, by using a proximal magnetic sensor and a distal magnetic sensor, an axial distance x between a proximal end and a distal end of a balloon of the balloon catheter, and/or an angle α between a central axis of the proximal end of the balloon and a central axis of the distal end of the balloon; wherein the proximal magnetic sensor is a magnetic localization sensor disposed at the proximal end of the balloon of the balloon catheter, and the distal magnetic sensor is a magnetic localization sensor disposed at the distal end of the balloon of the balloon catheter; and
based on a predefined first correspondence between the axial distance x and an axial contact pressure of the balloon catheter, and/or a predefined second correspondence between the angle α and a lateral contact pressure of the balloon catheter, determining an axial contact pressure and/or a lateral contact pressure by mapping the detected axial distance x and/or the detected angle α.

In an embodiment, the first correspondence is determined by a fitting formula, and/or the second correspondence is determined by a fitting formula.

In an embodiment, the first correspondence and/or the second correspondence are/is determined based on the average value of two or more sets of experimental data.

In an embodiment, the method further comprises: detecting the inflation pressure of the balloon catheter, and selecting a corresponding first correspondence and/or a corresponding second correspondence based on the detected inflation pressures.

In an embodiment, the inflation pressure is 1 ± 0.2 psi.

In an embodiment, the balloon catheter is inflated with a liquid medium.

In a second aspect, the present disclosure provides a balloon catheter ablation system.

A balloon catheter ablation system includes:
a balloon catheter, comprising a catheter shaft and a balloon electrode assembly, wherein the balloon electrode assembly is fixed at a distal end of the catheter shaft and includes a balloon that is configured to undergo axial compressive deformation and/or lateral deflection under pressure; a proximal magnetic sensor is disposed at a proximal end of the balloon, and a distal magnetic sensor is disposed at a distal end of the balloon, wherein the distal magnetic sensor is configured to move with the distal end of the balloon relative to the proximal magnetic sensor during axial deformation and/or lateral deflection; and
a processing unit configured to, based on a relative movement between the proximal magnetic sensor and the distal magnetic sensor, detect an axial distance x between the proximal and distal ends of the balloon and/or an angle α between a central axis of the proximal end of the balloon and a central axis of the distal end of the balloon, and based on a predefined first correspondence between the axial distance x and an axial contact pressure of the balloon catheter and/or a predefined second correspondence between the angle α and a lateral contact pressure of the balloon catheter, determine an axial contact pressure and/or a lateral contact pressure by mapping the detected axial distance x and/or the detected angle α.

In an embodiment, the axis of the proximal magnetic sensor is coincident with the central axis of the proximal end of the balloon.

In an embodiment, the proximal magnetic sensor is coaxially fixed to the distal end of the catheter shaft.

In an embodiment, the proximal magnetic sensor is provided with a relief through-hole extending in an axial direction of the catheter shaft; the balloon catheter includes an inflation tube for delivering inflation fluid to and from the balloon, and the inflation tube passes through the relief through-hole.

In an embodiment, the distal magnetic sensor includes a signal line, which is routed either through the relief through-hole or the inflation tube.

In an embodiment, the axis of the distal magnetic sensor is coincident with the central axis of the distal end of the balloon; and when the balloon catheter is in a free state, the axis of the distal magnetic sensor is coincident with the axis of the catheter shaft.

In an embodiment, the distal magnetic sensor includes a signal line, wherein the signal line is flexible and is configured to bend during compression and/or lateral deflection of the balloon.

In an embodiment, the signal line is provided with a cable sheath, and an end of the cable sheath is sealed to the distal magnetic sensor.

In an embodiment, the system further comprises a pressure sensor configured to detect the inflation pressure of the balloon, wherein the pressure sensor is connected to the processing unit.

In an embodiment, the system further comprises a handle connected to the proximal end of the catheter shaft; wherein an inner cavity of the balloon is in communication with the handle via the inflation tube, and wherein the pressure sensor is disposed within the handle and is in communication with the inflation tube.

The beneficial effects of the present disclosure are as follows:
According to the aforementioned method for detecting contact pressure of a balloon catheter, the axial distance x between the proximal and distal ends of the balloon and/or the angle α between the central axis of the proximal end and the central axis of the distal end of the balloon can be determined by using the proximal and distal magnetic sensors. Furthermore, based on a pre-obtained first correspondence between the axial distance x and the axial contact pressure of the balloon catheter and/or a pre-obtained second correspondence between the angle α and the lateral contact pressure of the balloon catheter, the actual axial contact pressure and/or lateral contact pressure value can be obtained by mapping the axial distance x and/or the angle α. Consequently, the magnetic localization sensors used for spatial positioning of the balloon catheter are shared for pressure detection, thereby eliminating the need for any additional dedicated contact pressure sensor. This avoids occupying extra space and increases costs.

According to the aforementioned balloon catheter ablation system, the balloon catheter employs a compliant balloon characterized by its ability to undergo axial compressive deformation and/or lateral deflection under pressure along the catheter. This characteristic works in synergy with the proximal magnetic sensor and the distal magnetic sensor to induce changes in the axial distance x and/or the angle α. Meanwhile, by utilizing the first and second correspondences pre-obtained by the processing unit, the corresponding contact pressure can be detected. This eliminates the need for any additional sensors dedicated to contact pressure detection, thereby avoiding the occupation of extra space and an increase in cost.

### BRIEF DESCRIPTION OF THE DRAWINGS

FIG. 1 is a perspective view of a balloon electrode assembly in an embodiment of the balloon catheter ablation system;
FIG. 2 is a front view of the balloon electrode assembly of FIG. 1;
FIG. 3 is a schematic diagram of the internal structure of the balloon electrode assembly of FIG. 1;
FIG. 4 is a schematic diagram of the balloon electrode assembly undergoing axial compressive deformation under pressure;
FIG. 5 is a fitting curve diagram of the axial distance x between the proximal end and the distal end versus the axial contact pressure under different inflation pressures;
FIG. 6 is a schematic diagram of the balloon electrode assembly undergoing lateral deflection under pressure relative to the balloon catheter;
FIG. 7 is a fitting curve diagram of the angle α between the central axis of the proximal end of the balloon and the central axis of the distal end of the balloon versus the lateral contact pressure under different inflation pressures; and
FIG. 8 is a flowchart of contact pressure detection in an embodiment.

### REFERENCE NUMERALS LIST

- 10: balloon
- 11: proximal end
- 12: distal end
- 13: equatorial position
- 20: insulating base
- 21: first electrode
- 22: second electrode
- 31: first trace
- 32: second trace
- 40: distal electrode
- 50: catheter shaft
- 51: inflation tube
- 61: proximal magnetic sensor
- 62: distal magnetic sensor
- 63: sensor protection sleeve
- 64: signal line
- 65: cable sheath
- 66: relief through-hole
- 70: heart

### DETAILED DESCRIPTION

Specific embodiments of the present disclosure will now be described in detail with reference to the accompanying drawings. Similar or related components in different embodiments are labeled with associated reference numerals. The following embodiments include detailed descriptions to facilitate understanding of the present disclosure. However, those skilled in the art will readily recognize that certain features may be omitted under specific circumstances or substituted by other components, materials, or methods. In some instances, certain operations related to the present disclosure are not explicitly described or illustrated herein. This intentional exclusion is intentional to avoid obscuring the core technical solutions of the present disclosure. For those skilled in the art, a complete understanding of these operations can be attained through the descriptions provided in this specification and general technical knowledge in the art.

Additionally, the features, operations, or characteristics described in the specification may be combined in any suitable manner to form various embodiments. Similarly, steps or actions in the method descriptions may be reordered or modified in ways that would be obvious to those skilled in the art. Therefore, the sequences presented in the specification and drawings are intended solely to clarify the description of specific embodiments and do not imply mandatory orderings, unless explicitly stated that a particular sequence is required.

The numerical designations assigned to components herein, such as "first", "second", and the like, are used solely to distinguish between the described objects and carry no implication of order or technical significance. The terms "connected" and "coupled" as used in this disclosure, unless otherwise specified, encompass both direct and indirect connections.

The terms "proximal" and "distal" as used herein are conventional in the medical field. For a medical instrument to be operated, the proximal end refers to the end closer to the operator of the instrument, while the distal end refers to the end farther from the operator. For example, in an ablation catheter, the balloon electrode assembly is connected to the distal end of the catheter shaft.

The present disclosure provides a method for detecting contact pressure of a balloon catheter and a balloon catheter ablation system. By utilizing the "compliance" (i.e., deformability) of the balloon of the balloon catheter, the axial distance x between its proximal and distal ends and/or the angle α between the central axis of the proximal end and the central axis of the distal end are detected via the proximal and distal magnetic sensors. Based on a predefined first correspondence between the axial distance x and the axial contact pressure of the balloon catheter, and/or a predefined second correspondence between the angle α and the lateral contact pressure of the balloon catheter, the actual axial and/or lateral contact pressure is calculated. This approach addresses the problems of excessive space occupation and high cost associated with using dedicated strain-gauge pressure sensors for contact pressure detection in the prior art. It thereby enables balloon-type ablation catheters equipped with an inflation tube to achieve contact pressure detection. This fulfills the need to guide physicians during ablation operations and, furthermore, allows for more efficient point-by-point ablation using the novel balloon-based pulsed field ablation catheter, ultimately improving surgical efficiency and quality.

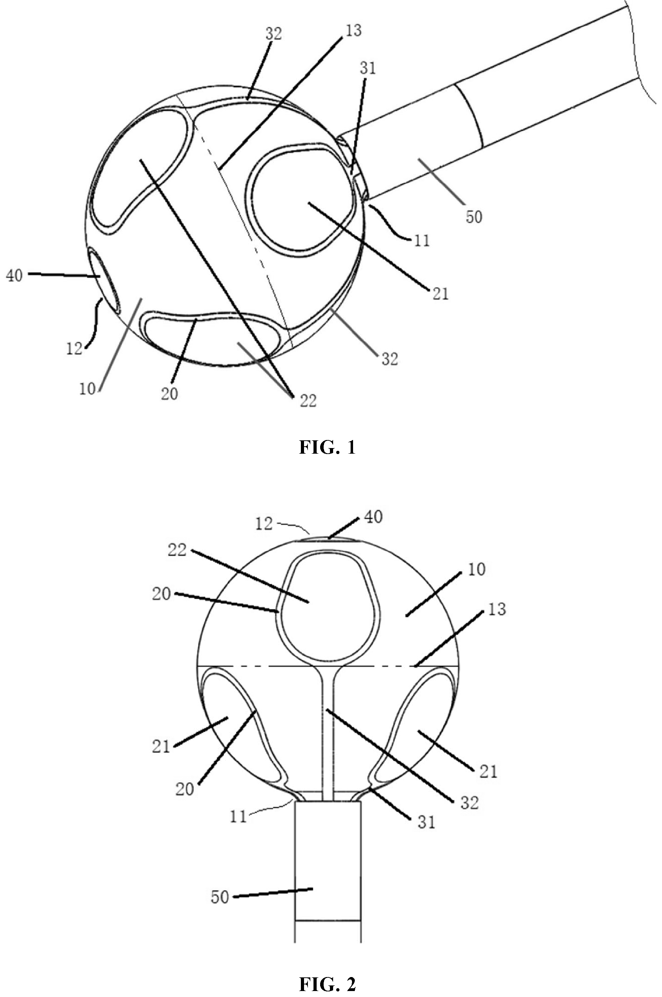

An embodiment of the balloon catheter ablation system of the present disclosure is described below:
The balloon catheter ablation system includes a balloon catheter, a handle, and a processing unit. Referring to FIGS. 1 to 3, the balloon catheter includes a catheter shaft 50 and a balloon electrode assembly. The catheter shaft 50 is configured for insertion into a site to be ablated. The balloon electrode assembly is fixed to the distal end of the catheter shaft 50 and includes a balloon 10, electrodes fixed on the surface of the balloon 10, a proximal magnetic sensor 61 disposed at the proximal end 11 of the balloon 10, and a distal magnetic sensor 62 disposed at the distal end 12 of the balloon 10. The balloon 10 is compliant and is configured to undergo axial compressive deformation and/or lateral deflection along the balloon catheter under pressure. The electrodes are configured to generate pulsed electric fields and deliver ablation energy. The handle (not shown), a common component of an ablation catheter, is connected to the proximal end 11 of the catheter shaft 50. It is for manipulating the balloon catheter, including extending, retracting, or deflecting the catheter shaft 50, and is configured to be hand-held by an operator. The processing unit is connected to the balloon electrode assembly to receive corresponding detection signals. Based on a relative movement between the proximal magnetic sensor 61 and the distal magnetic sensor 62, the processing unit detects an axial distance x between the proximal end 11 and the distal end 12 of the balloon 10 and/or an angle α between the central axis of the balloon's proximal end 11 and the central axis of the balloon's distal end 12. Then, based on a known first correspondence between the axial distance x and an axial contact pressure of the balloon catheter, and/or a known second correspondence between the angle α and a lateral contact pressure of the balloon catheter, the processing unit determines an actual axial contact pressure and/or lateral contact pressure by mapping the detected axial distance x and/or the detected angle α.

It should be noted that the balloon catheter ablation system may include an ablation console. The ablation console may have, for example, a display module, a power supply module for providing energy to the balloon catheter, and a control module for controlling the operation of the balloon catheter, and may also include the aforementioned processing unit. Of course, in some other embodiments, the processing unit may be independent of the ablation console, used solely for detecting the contact pressure of the balloon catheter. Furthermore, since magnetic localization sensors are commonly integral components of existing three-dimensional (3D) mapping systems, a 3D mapping system may also be used as the processing unit connected to the magnetic localization sensors, thereby completing the detection process. In an embodiment of the balloon electrode assembly, the electrodes on the surface of the balloon 10 are configured to include a first electrode 21 disposed on the proximal side and a second electrode 22 disposed on the distal side. The first electrode 21 and the second electrode 22 are arranged to be axially offset along the balloon 10 and circumferentially staggered. With this configuration, during ablation, depending on the orientation of the balloon electrode assembly, the first electrode 21 and the second electrode 22 can ablate individually, or both can ablate simultaneously when the equatorial position 13 of the balloon electrode assembly contacts the target tissue, thereby achieving a larger ablation area. Coupled with the pulsed field ablation method, which offers greater ablation depth, larger ablation lesions can be obtained, which is conducive to improving procedural efficiency. Specifically, the electrodes include at least one first electrode 21 and at least one second electrode 22. The first electrode 21 is offset toward the proximal side of the balloon 10, and the second electrode 22 is offset toward the distal side of the balloon 10. The first electrode 21 and the second electrode 22 are staggered along the circumferential direction of the balloon 10, where the circumferential direction of the balloon 10 refers to the direction encircling the line connecting the proximal end 11 and the distal end 12.

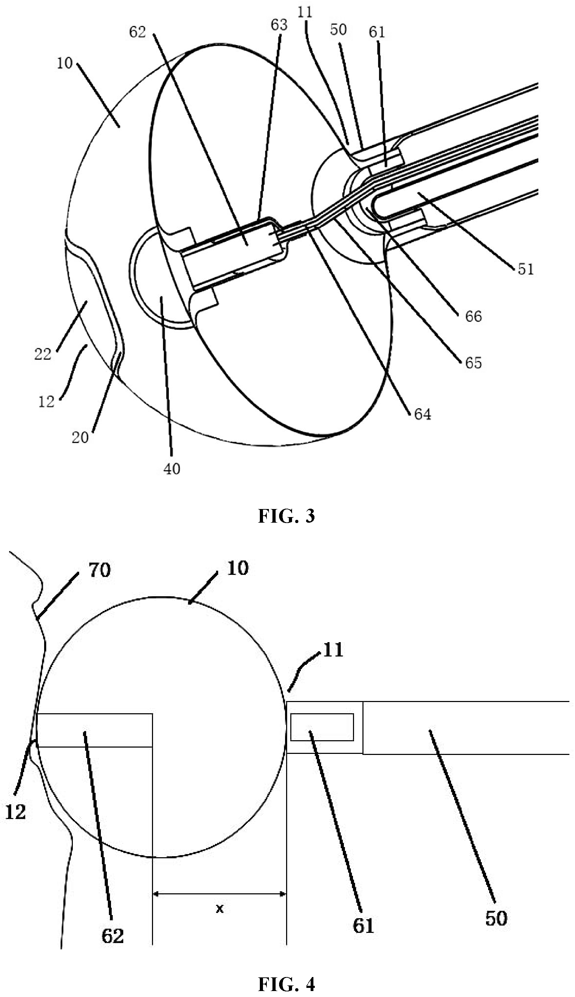

The balloon 10 can be inflated by gas or liquid, with no specific limitation imposed by the present disclosure. Moreover, the inflation and deflation of the balloon 10 can be accomplished using conventional means, for example, by delivering gas or liquid through a corresponding channel within the catheter shaft 50 to inflate it. As shown in FIG. 3, the corresponding channel within the catheter shaft 50 is the inflation tube 51. The inflation tube 51 allows inflation fluid to flow into and out of the balloon 10; and the inflation fluid may be physiological saline. It should be noted that the balloon 10 assumes a spherical shape upon inflation. As used herein, "spherical" may refer to a standard sphere, or to similar spheroidal shapes, such as an ellipsoid.

In a specific embodiment, the compliant material selected for the balloon 10 may be, for example, TPU or PEBAX. It should be noted that lateral deflection of the balloon catheter under pressure is not limited to being perpendicular to the axis of the balloon catheter. It may also occur at an oblique angle relative to the axis of the balloon catheter, and may correspond to a position on the side of the balloon 10 that is offset toward the proximal end 11 or the distal end 12. Owing to the compliant nature of the balloon material, the distal magnetic sensor 62 forms a free-floating structure. Thus, when the balloon 10 undergoes axial compressive deformation and/or lateral deflection under pressure, the distal magnetic sensor 62 can move relative to the proximal magnetic sensor 61 as the distal end 12 of the balloon displaces.

Unlike the thermal conduction mechanism of radiofrequency ablation, pulsed field ablation relies on pulsed electric fields for energy delivery. The electrodes affixed to the surface of the balloon 10 of the balloon electrode assembly are configured to generate the required electric field. These electrodes may be mounted on an insulating base 20 to form a flexible circuit, which is then affixed to the balloon 10. To route the electrical connections of the electrodes, the flexible circuit incorporates metal conductors for energy transmission, which, together with the corresponding insulating base 20, form conductive traces. In an embodiment, the balloon 10 of the balloon electrode assembly is a small balloon 10 with a diameter of 12 mm, and six flexible circuits (e.g., having an elongated, tapered shape resembling bean sprouts) are affixed to the surface of the balloon 10.

In an embodiment, and with reference to FIGS. 1 and 2, the first electrode 21 is offset toward the proximal side of the balloon 10, and the second electrode 22 is offset toward the distal side of the balloon 10. It should be noted that describing an electrode as being "offset toward the proximal side" is not intended to limit the electrode as being entirely located within the hemispherical region of the balloon 10 adjacent to the proximal end 11. For example, such an electrode may be partially located within the hemispherical region of the balloon 10 adjacent to the proximal end 11 and partially located within the hemispherical region of the balloon 10 adjacent to the distal end 12. However, in its entirety, the electrode is offset toward the hemispherical dividing line of the balloon 10. A similar description of being "offset toward the distal side" shall be construed in a similar manner, and will not be further described herein.

The aforementioned hemispherical dividing line may be regarded as the equatorial position 13 of the balloon 10, which is the position of maximum radial dimension when the balloon is in an inflated state and which extends circumferentially around the balloon 10. With reference to FIGS. 1 and 2, in an embodiment, the side of the first electrode 21 adjacent to the distal end 12 does not extend beyond the equatorial position 13, and the side of the second electrode 22 adjacent to the proximal end 11 does not extend beyond the equatorial position 13. In summary, in an embodiment, the first electrode 21 and the second electrode 22 are free of axial overlap relative to the balloon 10. This configuration ensures that when the balloon 10 is oriented at an axial oblique angle relative to the surface of the target ablation tissue during the ablation procedure, the first electrode 21 or the second electrode 22 that is adjacent to the tissue can achieve an increased ablation range and, consequently, a larger ablation surface area. Of course, in other embodiments, the first electrode 21 and the second electrode 22 may alternatively exhibit axial overlap relative to the balloon 10, even when arranged along the circumferential direction of the balloon 10. For example, the side of the first electrode 21 adjacent to the distal end 12 extends beyond the equatorial position 13, while the side of the second electrode 22 adjacent to the proximal end 11 is aligned with the equatorial position 13 or spaced apart from it. Alternatively, the side of the first electrode 21 adjacent to the distal end 12 is aligned with the equatorial position 13 or spaced apart from it, while the side of the second electrode 22 adjacent to the proximal end 11 extends beyond the equatorial position 13.

To further expand the ablation range, the width dimension of the first electrode 21 tapers upward from the proximal end 11 to the distal end 12, and the width of the second electrode 22 tapers upward from the distal end 12 to the proximal end 11. This tapering characteristic is not intended to require the first electrode 21 to have a uniform taper across its entire length, and the width variation may instead be limited to only a portion of the electrode. With reference to FIG. 2, in an embodiment, the edges of the first electrode 21 distal from the proximal end 11 and adjacent to the proximal end 11 are arcuate; the edges of the second electrode 22 distal from the distal end 12 and adjacent to the distal end 12 are also arcuate. Meanwhile, the edges connecting the arcuate edges at both ends of the first electrode 21 and the second electrode 22 are inclined straight edges, with a gradual width taper. This width tapering configuration is conducive to optimizing electric field distribution and expanding the ablation range.

In an embodiment, at least two first electrodes 21 and at least two second electrodes 22 are provided. In the circumferential direction of the balloon, one second electrode 22 is disposed between every two adjacent first electrodes 21, and one first electrode 21 is disposed between every two adjacent second electrodes 22. The first electrodes 21 and the second electrodes 22 thus form a cross-arranged configuration. With reference to FIGS. 3 and 4, in an embodiment, three first electrodes 21 and three second electrodes 22 are provided. It should be noted that the number of electrodes may be determined based on factors including the diameter of the balloon 10, ablation requirements, and the area of each electrode. The first electrodes 21 may have the same shape as the second electrodes 22, as shown in FIGS. 2-5, or may have a different shape. Additionally, at least two of the first electrodes 21 may have different shapes, and/or at least two of the second electrodes 22 may have different shapes.

Based on the cross-arranged configuration of the first electrodes 21 and the second electrodes 22, in an embodiment, the balloon electrode assembly includes a first trace 31 and a second trace 32. The first trace 31 is connected to the first electrode 21, and the second trace 32 is connected to the second electrode 22. At least a portion of the second trace 32 is arranged circumferentially parallel to the first electrode 21. This trace arrangement facilitates electrical energy delivery to the second electrode 22 while also providing a streamlined configuration. Preferably, the first trace 31 and the first electrode 21 may be centrally aligned, and the second trace 32 and the second electrode 22 may be centrally aligned. The proximal end 11 of each trace may be routed via a wire through the catheter shaft 50 of the ablation catheter to the handle of the ablation catheter.

When the balloon electrode assembly is performing an ablation procedure, with the longitudinal axis of the ablation catheter defined as the axial direction and the surface of the target ablation tissue modeled as an ideal plane, the contact modes of the balloon electrode assembly of the ablation catheter against the tissue can be simplified into three configurations, namely, the axial direction being perpendicular to the plane, the axial direction being inclined relative to the plane, and the axial direction being parallel to the plane. In a typical embodiment, the axial direction may form angles of 90°, 45°, and 0° with the plane, respectively. To ensure maximum contact between the electrodes and the tissue across all contact modes, in an embodiment, the distal end 12 of the balloon 10 does not protrude beyond the spherical profile of the balloon 10; that is, no structure protruding from the smooth contour is disposed at the distal end 12 of the balloon 10. This eliminates the risk of protrusions at the distal end 12 of the balloon 10 interfering with the orientation of the balloon electrode assembly.

In an embodiment, to enable additional ablation capabilities, the electrodes include a distal electrode 40 disposed at the distal end 12 of the balloon 10. The distal electrode 40 forms a distal cover configured to seal the opening formed at the distal end 12 of the balloon 10 during its manufacturing process. The distal cover may be fabricated from a metallic electrode material to form the distal electrode 40, which is electrically connected via an independent lead wire routed through the interior of the balloon 10. Specifically, the distal electrode 40 may be fabricated from 316 stainless steel or platinum-iridium alloy. Of course, in other embodiments, the distal end 12 of the balloon 10 may alternatively be configured as an insulating structure, for example, sealed with epoxy resin or other plastic injection-molded components.

To avoid the distal electrode 40 forming a protruding structure at the distal end 12 of the balloon 10, furthermore, in an embodiment, with reference to FIG. 3, the distal end 12 of the balloon 10 is configured with a concave recess, and the distal electrode 40 is disposed within the concave recess.

In operation, the balloon electrode assembly is coupled to the distal end 12 of the catheter and is advanced into a biological body through a sheath together with the catheter. For target ablation lesions, the orientation of the balloon electrode assembly may be adjusted by controlling the deflection of the catheter, allowing the balloon electrode assembly to abut against the tissue at varying angles, after which pulsed field ablation may be performed using the corresponding electrodes. Since the balloon electrode assembly of the present disclosure is capable of achieving an increased ablation range and depth, this helps reduce the number of ablation lesion on the heart 70, thereby enhancing procedural efficiency, alleviating patient discomfort, and reducing the procedural burden on physicians.

The foregoing description primarily sets forth the configuration of the balloon electrode assembly used for implementing pulse field ablation. The following discussion will focus on how to detect the contact pressure of the balloon electrode assembly.

Firstly, with respect to the proximal magnetic sensor 61 and distal magnetic sensor 62 relied upon for contact pressure detection, those skilled in the art will appreciate that the proximal magnetic sensor 61 and the distal magnetic sensor 62, as magnetic localization sensors, are commonly used positioning components in catheter systems. These magnetic localization sensors may be coils and may be positioned within a corresponding magnetic field during operation. The magnetic localization system can obtain the position of each magnetic localization sensor in the magnetic field based on the magnetic field conditions sensed by the magnetic localization sensors at different positions, with a typical positional resolution of 0.5-1.0 mm. As the positioning methodology using magnetic localization sensors is well-established in the art, detailed explanations are not provided herein.

In a specific embodiment, the proximal magnetic sensor 61 employs a commercially available 5DOF magnetic localization sensor, while the distal magnetic sensor 62 employs a commercially available 6DOF magnetic localization sensor. Essentially, a 6DOF magnetic localization sensor may be fabricated by packaging two 5DOF magnetic localization sensors at a fixed angle (e.g., 15°). In contrast to a 5DOF magnetic localization sensor, a 6DOF magnetic localization sensor is additionally capable of outputting raw angular data. Such raw angular data is not essential for the present disclosure. In some other embodiments, depending on detection requirements, both the proximal magnetic sensor 61 and the distal magnetic sensor 62 may employ 5DOF magnetic localization sensors, or alternatively, other types of magnetic localization sensors may be used, provided that they can detect the axial distance x between the proximal 11 and the distal 12 of the balloon 10, and/or the angle α between the central axis of the balloon's proximal 11 and that of its distal end 12.

In an embodiment, for ease of calculation, the axis of the distal magnetic sensor 62 coincides with the central axis of the distal 12 of the balloon 10. When the balloon catheter is in a free state, the axis of the distal magnetic sensor 62 coincides with the axis of the catheter shaft 50. In a specific embodiment, the distal magnetic sensor 62 is a solid cylinder with typical dimensions of 1.8×9.0 mm and may be affixed to the inner side of the distal cover of the balloon 10, arranged coaxially with the central axis of the distal 12 of the balloon 10. Given that the balloon 10 may need to be filled with liquid, to protect the distal magnetic sensor 62, a sensor protection sleeve 63 is disposed over the exterior of the distal magnetic sensor 62 so as to fully enclose the distal magnetic sensor 62.

In an embodiment, the distal magnetic sensor 62 includes a signal line 64, which is a flexible line configured to flex in response to compressive deformation and/or lateral deflection of the balloon 10. To protect the signal line 64 of the distal magnetic sensor 62, a cable sheath 65 may be disposed over the exterior of the signal line 64. A distal end of the cable sheath 65 is sealed to the distal magnetic sensor 62; specifically, this sealed joint may be enclosed and sealed by the sensor protection sleeve 63. Both the signal line 64 and the cable sheath 65 may be fabricated from flexible materials, ensuring that the movement of the distal magnetic sensor 62 is not restricted by the signal line 64 and the cable sheath 65. Of course, in other embodiments, the distal magnetic sensor 62 may employ a wireless signal transmission configuration, and the proximal magnetic sensor 61 may also employ a wireless signal transmission configuration, in which case the signal line 64 is not required.

Similar to the distal magnetic sensor 62, for ease of calculation, the axis of the proximal magnetic sensor 61 coincides with the central axis of the proximal 11 of the balloon 10. Furthermore, in a specific embodiment, the proximal magnetic sensor 61 is fixed coaxially to the distal end of the catheter shaft 50, a configuration that conserves internal space within the balloon 10 and facilitates the stable placement of the proximal magnetic sensor 61. Of course, in some other embodiments, the proximal magnetic sensor 61 may alternatively be at least partially fixed to the balloon 10, while still being positioned at the proximal end of the balloon 10.

In an embodiment, since the inflation tube 51 needs to be in communication with the inner cavity of the balloon 10 to enable the expansion and collapse of the balloon 10, to facilitate the routing of the inflation tube 51, with reference to FIG. 3, the proximal magnetic sensor 61 is a hollow cylindrical coil. The hollow structure forms a relief through-hole 66 extending along the axial direction of the sensor 61, which is coaxial with the catheter shaft 50. The inflation tube 51 of the balloon catheter is routed through the relief through-hole 66. With reference to FIG. 3, the distal end 12 of the inflation tube 51 is located at the proximal end 11 of the balloon 10, which is configured to allow inflation fluid to flow into and out of the balloon 10. In other embodiments, the relief through-hole 66 may alternatively be offset radially to one side of the catheter shaft 50.

In an embodiment, the proximal magnetic sensor 61 may alternatively be a miniature solid cylindrical sensor, with a typical size of 0.45×6.3 mm.

Meanwhile, with reference to FIG. 3, the signal line 64 of the distal magnetic sensor 62 is routed parallel to the inflation tube 51 and may be routed through the relief through-hole 66. In some other embodiments, the signal line 64 may alternatively be routed through the interior of the inflation tube 51.

The primary factors influencing pressure value fitting further include the initial hydraulic pressure inside the balloon 10. Therefore, in one embodiment, the balloon catheter ablation system further includes a pressure sensor, which is connected to the processing unit.

Given the limited internal space within the balloon 10, in one embodiment, the handle is connected to the proximal end 11 of the catheter shaft 50, and the inner cavity of the balloon 10 is in fluid communication with the handle via the inflation tube 51, while the pressure sensor is disposed within the handle and is in fluid communication with the inflation tube 51. Of course, in some other embodiments, the pressure sensor may alternatively be disposed within the balloon 10.

Based on the structure of the balloon catheter described above, the following describes a method for detecting the contact pressure of a balloon catheter.

In general, prior to performing actual contact pressure detection for the balloon catheter, a first correspondence between the axial distance x and the axial contact pressure of the balloon catheter, and/or a second correspondence between the angle α and the lateral contact pressure of the balloon catheter, is first established experimentally. The axial distance x and the angle α may be obtained by the magnetic localization system via the proximal magnetic sensor 61 and the distal magnetic sensor 62, whereas the axial and lateral contact pressures may be detected via sensors. For example, temporary placement of a sensor on the target ablation tissue or on the balloon 10 enables acquisition of the axial and/or lateral contact pressure values. In an embodiment, to improve data accuracy, the first correspondence is determined from the average of two or more sets of experimental data, and/or the second correspondence is likewise determined from the average of two or more sets of experimental data, with each correspondence corresponding to different samples as shown in the table below.

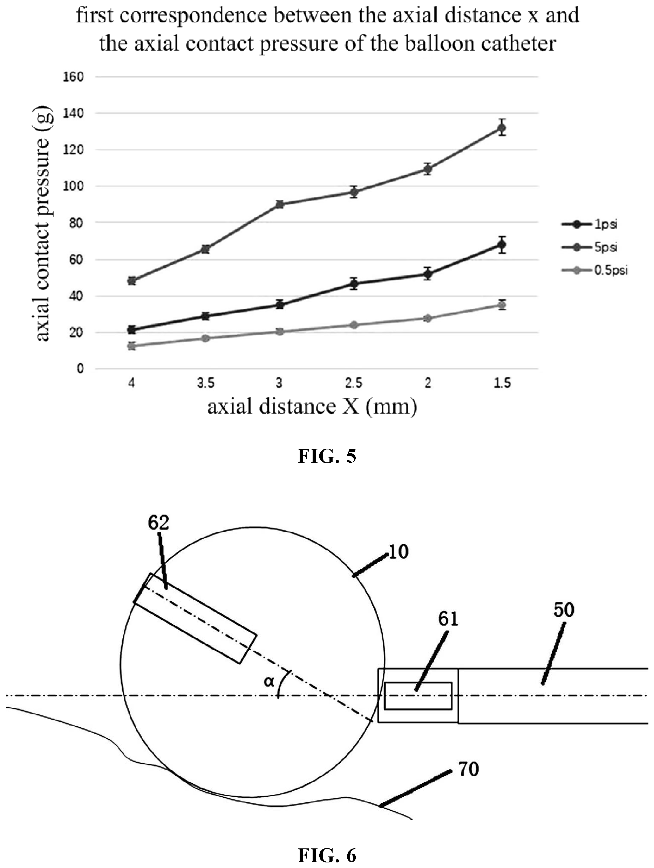

For example, for the first correspondence between the axial distance x and the axial contact pressure of the balloon catheter, the initial axial distance x is 4.0 mm. When the balloon catheter abuts the tissue perpendicularly, the distance x between the two magnetic localization sensors is compressed. Application of a series of axial contact pressures yields the fitting relationship between the reduction in axial contact x and the axial contact pressure, as shown in Table 1 and FIG. 5. Of course, depending on precision requirements, the incremental gradient of axial contact pressure change and the number of samples used in the experiments may be adjusted as needed.

It should be noted that the fitting curve is affected by the initial inflation pressure. When the initial inflation pressure is high, for example, 5 psi, the balloon 10 requires a higher force to generate strain, while when the initial inflation pressure is low, for example, 0.5 psi, the balloon requires a lower force to generate strain. When detecting the actual balloon catheter contact pressure, the corresponding fitting relationship may be selected based on the actual inflation pressure. In one specific embodiment, the inflation pressure is 1 ± 0.2 psi. Additionally, the filling medium used to inflate the balloon 10 may be either a gas or a liquid. Given that gas has a higher compression ratio, the pressure required to generate strain is lower. The filling medium utilized in this experiment is a liquid, and the inflation pressures are 0.5 psi, 1 psi, and 5 psi, respectively. The corresponding experimental data are shown in Table 1.

**Table 1**

| Axial Distance X (mm) (Initial Inflation Pressure 1 psi) | Sample 1 | Sample 2 | Sample 3 | Sample 4 | Average | Standard Deviation |
|---|---|---|---|---|---|---|
| 4 | 18 | 21 | 22 | 24 | 21.25 | 2.5 |
| 3.5 | 28 | 29 | 30 | 27 | 28.5 | 1.290994 |
| 3 | 38 | 33 | 36 | 34 | 35.25 | 2.217356 |
| 2.5 | 45 | 47 | 46 | 49 | 46.75 | 1.707825 |
| 2 | 51 | 53 | 53 | 51 | 52 | 1.154701 |
| 1.5 | 69 | 70 | 67 | 66 | 68 | 1.825742 |

| Axial Distance X (mm) (Initial Inflation Pressure 5 psi) | Sample 1 | Sample 2 | Sample 3 | Sample 4 | Average | Standard Deviation |
|---|---|---|---|---|---|---|
| 4 | 45 | 48 | 50 | 49 | 48 | 2.160247 |
| 3.5 | 68 | 66 | 63 | 65 | 65.5 | 2.081666 |
| 3 | 88 | 89 | 93 | 91 | 90.25 | 2.217356 |
| 2.5 | 96 | 93 | 99 | 100 | 97 | 3.162278 |
| 2 | 113 | 108 | 112 | 106 | 109.75 | 3.304038 |
| 1.5 | 133 | 135 | 136 | 126 | 132.5 | 4.50925 |

| Axial Distance X (mm) (Initial Inflation Pressure 0.5 psi) | Sample 1 | Sample 2 | Sample 3 | Sample 4 | Average | Standard Deviation |
|---|---|---|---|---|---|---|
| 4 | 10 | 12 | 13 | 15 | 12.5 | 2.081666 |
| 3.5 | 16 | 16 | 17 | 18 | 16.75 | 0.957427 |
| 3 | 20 | 19 | 21 | 22 | 20.5 | 1.290994 |
| 2.5 | 23 | 23 | 24 | 25 | 23.75 | 0.957427 |
| 2 | 26 | 28 | 27 | 29 | 27.5 | 1.290994 |
| 1.5 | 34 | 38 | 32 | 36 | 35 | 2.581989 |

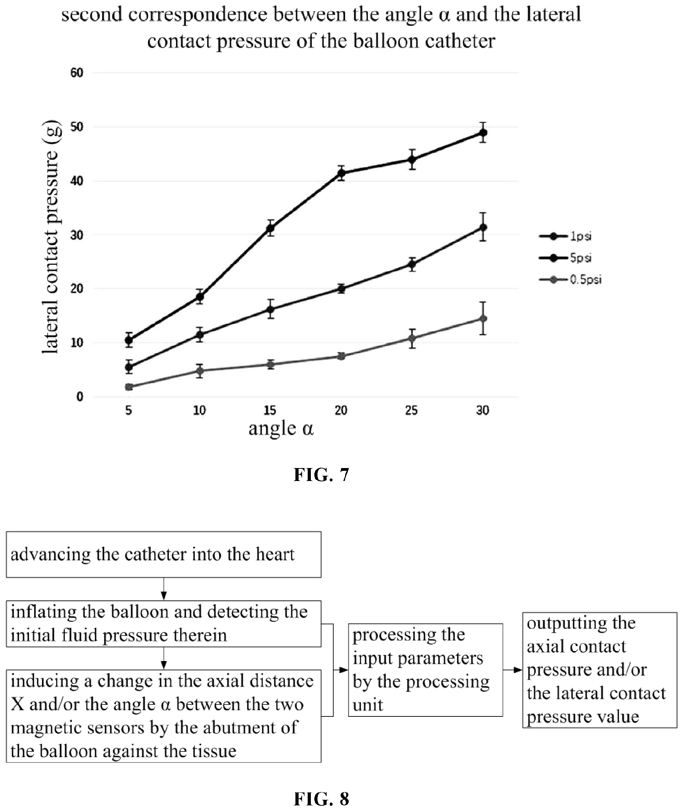

For the second correspondence between the angle α and the lateral contact pressure of the balloon catheter, for example, when the initial angle α is 0° and the balloon catheter abuts the tissue laterally, the angle α between the central axis of the proximal end 11 of the balloon 10 and the central axis of the distal end 12 of the balloon 10, that is, the angle α between the two magnetic localization sensors, increases. Application of a series of lateral contact pressures yields the fitting relationship between the increase in angle α and the lateral contact pressure, as shown in Table 2 and FIG. 7. Of course, depending on precision requirements, the incremental gradient of lateral contact pressure and the number of samples used in the experiments may be adjusted as needed.

**Table 2**

| Angle (α°) (Initial Inflation Pressure 1 psi) | Sample 1 | Sample 2 | Sample 3 | Sample 4 | Average | Standard Deviation |
|---|---|---|---|---|---|---|
| 5 | 5 | 4 | 6 | 7 | 5.5 | 1.290994 |
| 10 | 12 | 10 | 13 | 11 | 11.5 | 1.290994 |
| 15 | 14 | 16 | 17 | 18 | 16.25 | 1.707825 |
| 20 | 20 | 19 | 20 | 21 | 20 | 0.816497 |
| 25 | 26 | 23 | 25 | 24 | 24.5 | 1.290994 |
| 30 | 35 | 30 | 32 | 29 | 31.5 | 2.645751 |

| Angle (α°) (Initial Inflation Pressure 5 psi) | Sample 1 | Sample 2 | Sample 3 | Sample 4 | Average | Standard Deviation |
|---|---|---|---|---|---|---|
| 5 | 10 | 9 | 11 | 12 | 10.5 | 1.290994 |
| 10 | 18 | 17 | 20 | 19 | 18.5 | 1.290994 |
| 15 | 33 | 32 | 30 | 30 | 31.25 | 1.5 |
| 20 | 40 | 42 | 43 | 41 | 41.5 | 1.290994 |
| 25 | 42 | 43 | 45 | 46 | 44 | 1.825742 |
| 30 | 47 | 48 | 51 | 50 | 49 | 1.825742 |

| Angle (α°) (Initial Inflation Pressure 0.5 psi) | Sample 1 | Sample 2 | Sample 3 | Sample 4 | Average | Standard Deviation |
|---|---|---|---|---|---|---|
| 5 | 2 | 2 | 1 | 2 | 1.75 | 0.5 |
| 10 | 3 | 5 | 6 | 5 | 4.75 | 1.258306 |
| 15 | 5 | 6 | 6 | 7 | 6 | 0.816497 |
| 20 | 7 | 7 | 8 | 8 | 7.5 | 0.57735 |
| 25 | 10 | 11 | 13 | 9 | 10.75 | 1.707825 |
| 30 | 12 | 12 | 16 | 18 | 14.5 | 3 |

Based on the above data in Table 1 and Table 2, the fitting curves shown in FIG. 5 and FIG. 7 are obtained, with the fitting formulas generated concurrently. The fitting curves and formulas may be stored in a processing unit, such as the balloon catheter ablation system, a standalone processing unit, and/or a three-dimensional mapping system. Those skilled in the art will appreciate that the fitting curves and formulas may be generated by a system from the experimental data.

During actual contact pressure detection, the balloon catheter ablation system tracks changes in the axial distance x and/or the angle α, and in conjunction with the fitting formulas, provides a physician with the actual axial and/or lateral contact pressure values.

An embodiment of a method for detecting contact pressure of a balloon catheter according to the present disclosure is described below:
With reference to FIG. 8, the method for detecting balloon catheter contact pressure includes the following steps:
advance an ablation catheter into the heart;
inflate the balloon and detect the inflation pressure;
when the inflated balloon catheter abuts the target ablation tissue and undergoes axial compressive deformation and/or lateral deflection, detect the axial distance x and/or the angle α between the proximal end 11 and the distal end 12 of the balloon 10 via the proximal magnetic sensor 61 and the distal magnetic sensor 62; the proximal magnetic sensor 61 is a magnetic localization sensor disposed at the proximal end 11 of the balloon 10 of the balloon catheter, and the distal magnetic sensor 62 is a magnetic localization sensor disposed at the distal end 12 of the balloon 10 of the balloon catheter; and
based on a predefined first correspondence between the axial distance x and the axial contact pressure of the balloon catheter and/or a predefined second correspondence between the angle α and the lateral contact pressure of the balloon catheter, derive the actual axial and/or lateral contact pressure values by mapping the measured axial distance x and/or the angle α.

It should be noted that the "filled balloon catheter" mentioned above refers to a catheter that meets predefined ablation requirements, and does not limit the filling degree of the balloon 10 of the balloon catheter. For example, inflation pressures of 0.5 psi, 1 psi, and 5 psi may correspond to different filling degrees.

In some other embodiments, the axial contact pressure may be obtained by detecting only the axial distance x, and the lateral contact pressure may be obtained by detecting only the angle α. If the volume of the balloon 10 is fixed and the filling medium used is a fixed volume, the initial inflation pressure is a fixed value, and the step of detecting the initial pressure within the balloon may be omitted, with contact pressure detection performed directly. Of course, in such cases, the system should also match the corresponding initial pressure to map the corresponding contact pressure values.

In some other embodiments, corresponding to the various embodiments of the aforementioned balloon catheter ablation system, corresponding methods for detecting balloon catheter contact pressure may be employed. For example, this may involve using different inflation pressures or different filling media.

The specific examples employed above to illustrate the present disclosure are for the purpose of facilitating understanding and are not intended to limit the disclosure. Those skilled in the art to which the present disclosure pertains, based on the concepts of the present disclosure, may make a number of simple deductions, variations, or substitutions.

## Claims

1. A method for detecting contact pressure of a balloon catheter, comprising:
when an inflated balloon catheter contacts a tissue to be ablated and undergoes axial compressive deformation and/or lateral deflection, detecting, by using a proximal magnetic sensor and a distal magnetic sensor, an axial distance x between a proximal end and a distal end of a balloon of the balloon catheter, and/or an angle α between a central axis of the proximal end of the balloon and a central axis of the distal end of the balloon; wherein the proximal magnetic sensor is a magnetic localization sensor disposed at the proximal end of the balloon of the balloon catheter, and the distal magnetic sensor is a magnetic localization sensor disposed at the distal end of the balloon of the balloon catheter; and
based on a predefined first correspondence between the axial distance x and an axial contact pressure of the balloon catheter, and/or a predefined second correspondence between the angle α and a lateral contact pressure of the balloon catheter, determining an axial contact pressure and/or a lateral contact pressure by mapping the detected axial distance x and/or the detected angle α.

2. The method according to claim 1, wherein the first correspondence is determined by a fitting formula, and/or the second correspondence is determined by a fitting formula.

3. The method according to claim 1 or 2, wherein the first correspondence and/or the second correspondence is determined based on an average value of two or more sets of experimental data.

4. The method according to claim 1 or 2, further comprising: detecting an inflation pressure of the balloon catheter; and selecting a corresponding first correspondence and/or a corresponding second correspondence based on a detected inflation pressure.

5. The method according to claim 4, wherein the inflation pressure is 1 ± 0.2 psi.

6. The method according to claim 1 or 2, wherein the balloon catheter is inflated with a liquid medium.

7. A balloon catheter ablation system, comprising:
a balloon catheter, comprising a catheter shaft and a balloon electrode assembly, wherein the balloon electrode assembly is fixed at a distal end of the catheter shaft and includes a balloon that is configured to undergo axial compressive deformation and/or lateral deflection under pressure; a proximal magnetic sensor is disposed at a proximal end of the balloon, and a distal magnetic sensor is disposed at a distal end of the balloon, wherein the distal magnetic sensor is configured to move with the distal end of the balloon relative to the proximal magnetic sensor during axial deformation and/or lateral deflection; and
a processing unit configured to, based on a relative movement between the proximal magnetic sensor and the distal magnetic sensor, detect an axial distance x between the proximal and distal ends of the balloon and/or an angle α between a central axis of the proximal end of the balloon and a central axis of the distal end of the balloon, and based on a predefined first correspondence between the axial distance x and an axial contact pressure of the balloon catheter and/or a predefined second correspondence between the angle α and a lateral contact pressure of the balloon catheter, determine an axial contact pressure and/or a lateral contact pressure by mapping the detected axial distance x and/or the detected angle α.

8. The system according to claim 7, wherein an axis of the proximal magnetic sensor is coincident with the central axis of the proximal end of the balloon.

9. The system according to claim 8, wherein the proximal magnetic sensor is coaxially fixed to the distal end of the catheter shaft.

10. The system according to claim 8, wherein the proximal magnetic sensor is provided with a relief through-hole extending in an axial direction of the catheter shaft; the balloon catheter includes an inflation tube for delivering inflation fluid to and from the balloon, and the inflation tube passes through the relief through-hole.

11. The system according to claim 10, wherein the distal magnetic sensor comprises a signal line routed through the relief through-hole or the inflation tube.

12. The system according to claim 7, wherein an axis of the distal magnetic sensor is coincident with the central axis of the distal end of the balloon; and when the balloon catheter is in a free state, the axis of the distal magnetic sensor is coincident with an axis of the catheter shaft.

13. The system according to any one of claims 7 to 12, wherein the distal magnetic sensor comprises a signal line, wherein the signal line is flexible and is configured to bend during compression and/or lateral deflection of the balloon.

14. The system according to claim 13, wherein the signal line is provided with a cable sheath, and an end of the cable sheath is sealed to the distal magnetic sensor.

15. The system according to any one of claims 7 to 12, further comprising a pressure sensor configured to detect an inflation pressure of the balloon, wherein the pressure sensor is connected to the processing unit.

16. The system according to claim 15, further comprising a handle connected to a proximal end of the catheter shaft; wherein an inner cavity of the balloon is in communication with the handle via the inflation tube, and wherein the pressure sensor is disposed within the handle and is in communication with the inflation tube.
